# FASCICULE DE BREVET EUROPEEN

(11) **EP 0 992 502 B1**
(45) Date de publication et mention de la délivrance du brevet: **26.06.2002**
(21) Numéro de dépôt: 99402433.9
(22) Date de dépôt: 05.10.1999
(51) Int. Cl.: C07D 405/14, C07D 217/04, A61K 31/473

(54) **Nouveaux dérivés de la 2-/3-phényl-2-propényl/-1,2,3,4-tétrahydro-isoquinoléine, leur procédé de préparation et leur application comme fongicides**
2-(3-Phenyl-2-propenyl)-1,2,3,4-tetrahydro-isochinolin-derivate, Verfahren zu ihrer Herstellung und ihre Verwendung als Fungizide
2-(3-Phenyl-2-propenyl)-1,2,3,4-tetrahydro-isochinolin derivatives, process for their preparation and their use as fungicides

(30) Priorité: 06.10.1998 FR 9812483; 26.08.1999 FR 9910811
(43) Date de publication de la demande: 12.04.2000
(73) Titulaire: Aventis Pharma S.A., 92160 Antony (FR)
(72) Inventeur: Babin, Didier, 78180 Montigny (FR); Braham, Abdel Karim, 93200 Saint Denis (FR); Hawser, Stephen, 28922 Verbagna (IT); Islam, Khalid, Trecallo Como 22100 (IT)

(56) Documents cités:
- EP-A- 0 050 298
- EP-A- 0 121 753
- US-A- 2 813 872

## Description

La présente invention concerne de nouveaux dérivés de la 2-[3-phényl-2-propényl]-1,2,3,4-tétrahydro-isoquinoléine, leur procédé de préparation et leur application comme fongicides.

Le document EP-A-0 121 753 aussi décrit des dérivés de 1,2,3,4-tétrahydroisoquinoléine ayant une activité antifongique.

L'invention a pour objet sous toutes les formes stéréoisomères possibles ainsi que leurs mélanges, les composés de formule (I) 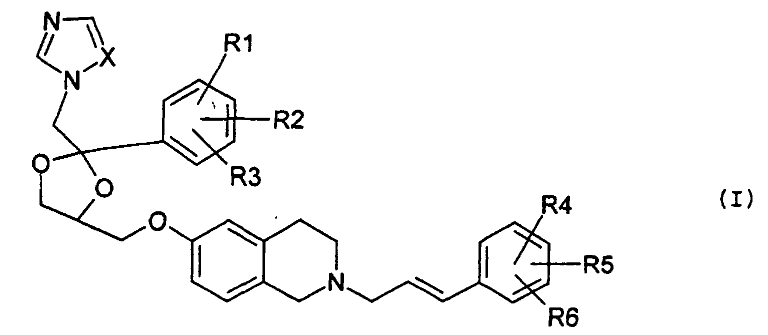 dans laquelle X représente un atome d'azote ou un radical -CH =, R1, R2, R3, R4, R5, R6 identiques ou différents en position quelconque sur les cycles qui les portent représentent un atome d'hydrogène, un atome d'halogène, un radical alkyle, O-alkyle, 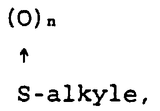 alkényle, O-alkényle, 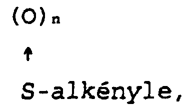 alkynyle, O-alkynyle, 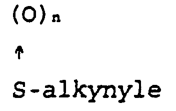 renfermant jusqu'à 8 atomes de carbone, éventuellement substitué par un ou plusieurs atomes d'halogène, n représentant le nombre 0, 1 ou 2, ou représentent un radical NO₂, NH₂ ou C≡N, R1, R2, R3, R4, R5 et R6 pouvant former des cycles deux à deux, ainsi que leurs sels d'addition ou avec les acides.

Les produits préférés sont les produits de structure cis au niveau du cycle dioxolane.

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, trifluoroacétique, propionique, benzoïque, citrique, maléique, fuma-rique, succinique, tartrique, alcane-sulfoniques, tels que les acides méthane ou éthane sulfoniques, arylsulfoniques tels que les acides benzène ou paratoluènesulfoniques.

Dans la définition des substituants,
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, cyclobutyle, cyclopentyle ou cyclohexyle,
- l'halogène est de préférence le fluor ou le chlore, ou le brome,

L'invention a plus particulièrement pour objet les composés de formule (IA) 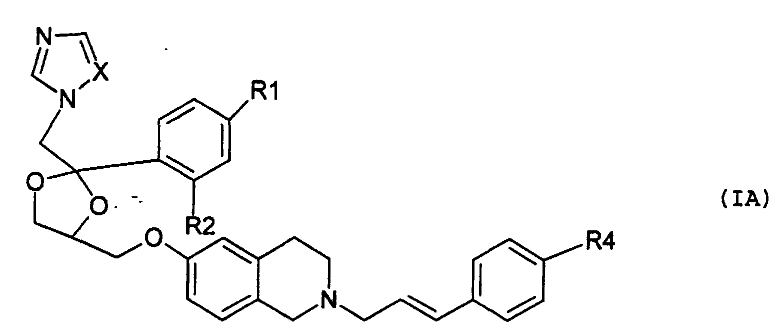 dans lesquels X, R1 R2 et R4 conservent leur signification précédente.

L'invention a tout spécialement pour objet les composés de formule (I) et (IA) dans lesquels X représente un radical -CH=, ceux dans lesquels R1 et R2 représentent chacun un atome d'halogène et ceux dans lesquels R4 représente un atome d'halogène.

L'invention a tout spécialement pour objet les composés de formule (I) dans lesquels R1, R2 et R4 représentent un atome de chlore.

Parmi les composés préférés de l'invention, on peut citer en particulier le composé de l'exemple 1 et en particulier son diastéréoisomère cis (+) décrit à l'exemple 2.

Les composés de formule (I) présentent d'intéressantes propriétés antifongiques ; ils sont notamment actifs sur Candida albicans et autres Candida comme Candida glabrata, krusei, tropicalis, pseudotropicalis et parapsilosis, sur Aspergillus fumigatus, Aspergillus flavus, Aspergillus niger Cryptococcus néoformans, Microsporum canis, Trichophyton rubrun, Trichophyton mentagrophyte.

Les composés de formule (I) peuvent être utilisés en tant que médicaments chez l'homme ou l'animal, pour lutter notamment contre les candidoses digestives, urinaires, vaginales ou cutanées, les cryptococcoses, par exemple les cryptococcoses neuroméningées, pulmonaires ou cutanées, les aspergilloses bronchopulmonaires et pulmonaires et les aspergilloses invasives de l'immunodéprimé.

Les composés de l'invention peuvent être utilisés également dans la prévention des affections mycosiques chez les déprimés immunitaires congénitaux ou acquis.

Les composés de l'invention ne sont pas limités à une utilisation pharmaceutique, ils peuvent être également utilisés comme fongicides dans d'autres domaines que pharmaceutiques.

L'invention a donc pour objet à titre de composés antifongiques, les composés de formule (I).

L'invention a également pour objet les composés de formule (I), à titre de médicaments.

L'invention a tout particulièrement pour objet les compositions pharmaceutiques renfermant comme principe actif au moins un composé de formule (I) ou l'un de ses sels.

Ces compositions peuvent être administrées par voie buccale, rectale, parentérale ou par voie locale en application topique sur la peau et les muqueuses, mais la voie préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les cyclodextrines, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 1.

L'invention a également pour objet un procédé de préparation, caractérisé en ce que l'on soumet un composé de formule (II) 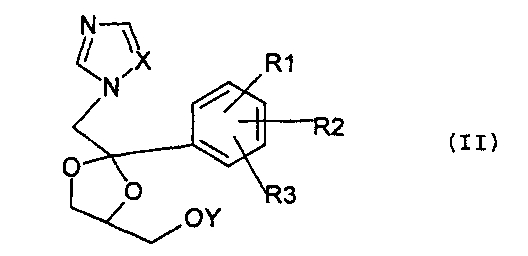 dans laquelle :
Y représente un radical mésyle ou tosyle
et les autres substituants conservent leur signification précédente à l'action d'un composé de formule (III) 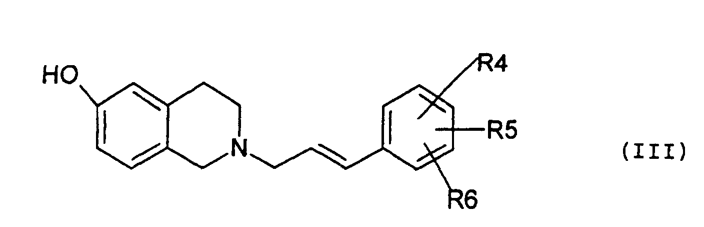
dans laquelle les différents substituants conservent leur précédente signification, pour obtenir le composé de formule (I) correspondant.

Les produits de formule (II) utilisés comme produits de départ sont des produits connus d'une façon générale et qui peuvent être préparés selon le procédé indiqué dans J. Med. Chem 1979 22(8)1003.

Les produits de formule (III) sont des produits nouveaux et sont en eux-mêmes un objet de la présente invention.

L'invention a tout spécialement pour objet le composé de formule (III) dont la préparation est donnée ci-après dans la partie expérimentale dont la préparation peut-être résumée comme suit :

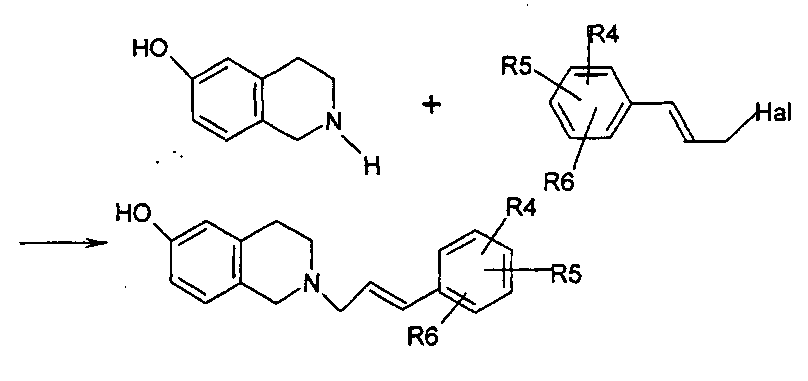

Les produits de formule (I) ainsi préparés peuvent être dédoublés en utilisant les procédés usuels.

L'invention a également pour objet un procédé de préparation des composés de formule (I) caractérisé en ce que l'on soumet un composé de formule (IV) : 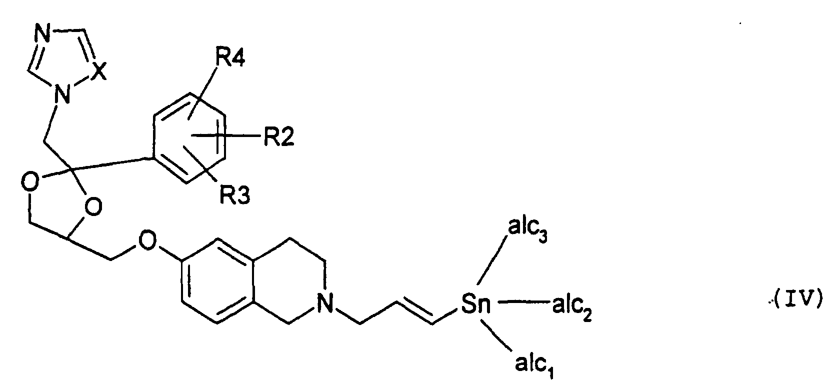 dans laquelle alc1, alc2 et alc3 représentent un radical alkyle renfermant jusqu'à 8 atomes de carbone, R1, R2, R3 et X conservent leur signification précédente, à l'action d'un composé de formule (V) : 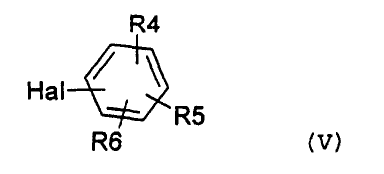 dans laquelle R4, R5 et R6 conservent leur signification précédente et Hal représente un atome d'halogène, en particulier un atome de brome ou d'iode, pour obtenir le composé formule (I) correspondant.

Les composés de formule (IV) utilisés comme produits de départ sont des produits nouveaux et sont en eux-mêmes un objet de l'invention.

Les exemples suivants illustrent l'invention sans toutefois la limiter.

### Préparation 1 : (E)-2-[3-(4-chlorophényl)-2-propényl]-1,2,3,4-tétrahydro-6-isoquinolinol.

On agite pendant 36 heures à 25°C un mélange de 2 g de 1,2,3,4- tétrahydro-6-isoquinolinol et 2,5 g de (E)-1-chloro-4-[3-chloro-1-propényl]-benzène, 2 g de carbonate de potassium et 50 ml de DMF. On chasse le DMF sous pression réduite. On reprend le résidu dans un mélange de chlorure de méthylène et d'eau. On filtre et obtient 2,5 g de produit recherché. F = 233-234°C.

### Exemple 1 : Cis-(±)-2-[3-(4-chlorophényl)-2-(E)-propényl]-6-[[2-(2,4-dichlorophényl)-2-(1H-imidazol-1-ylméthyl)-1,3-dioxolan-4-yl]méthoxy]-1,2,3,4-tétrahydro-isoquinoline.

On porte au reflux pendant 3,5 heures un mélange de 2 g du produit de la préparation 1, 3,1 g de méthanesulfonate de Cis-(+-)-2-(2,4-dichlorophényl)-2-(1H-imidazol-1-ylméthyl)-1,3-dioxolane-4-méthanol, 50 ml de toluène, 4 ml d'une solution d'hydroxyde de sodium à 50 %, 300 ml de chlorure de tribenzylammonium (C₆H₅CH₂ Et₃N⁺ Cl⁻). On refroidit. On sépare la couche toluénique, extrait la couche aqueuse avec du toluène, combine les deux couches toluéniques, sèche, filtre et évapore. On recristallise le produit obtenu dans un mélange d'éther éthylique et d'acétate d'éthyle. On obtient 3,2 g de produit recherché fondant à 127 ~ 129°C.
Analyse
C 62,91 %
H = 4,95 %
N = 6,88 %
Cl = 17,41 %
O₃ = 7,86 %

### Exemple 2 : Cis-(+)-2-[3-(4-chlorophényl)-2-(E)-propényl]-6-[[2-(2,4-dichlorophényl)-2-(1H-imidazol-1-ylméthyl)-1,3-dioxolan-4-yl]méthoxy]-1,2,3,4-tétrahydro-isoquinoline.

On chauffe à 90°C pendant 3 heures et 30 minutes un mélange de 480 mg de 4-méthylphénylsulfonate de cis(+)-2-(2,4-dichlorophényl)-2-(1H-imidazol-1-ylméthyl)-1,3-dioxolane-4-méthanol, 300 mg du produit de la préparation 1,276 mg de carbonate de potassium et 10 cm³ de DMF. On maintient sous agitation à la température ambiante pendant une nuit. On filtre, rince avec du DMF, amène le filtrat à sec à 40°C sous pression réduite. On reprend le résidu avec du chlorure de méthylène et de l'eau (50/50), agite et décante. On extrait la phase aqueuse avec du chlorure de méthylène, rassemble les phases organiques, les sèche, filtre et amène à sec à 35°C. On purifie le produit obtenu par chromatographie sur silice en éluant avec un mélange acétate d'éthyle méthanol 93/7. On obtient le produit brut que l'on triture dans 4 cm³ d'un mélange éther éthylique acétate d'éthyle 9/1, essore, lave à l'éther éthylique. On sèche et obtient 338 mg de produit que l'on purifie par CCP en éluant avec le mélange acétate d'éthyle, méthanol (93/7). On obtient 332 mg de produit. F = 110°C.
α_{D} = + 12° (c = 1% méthanol)

### Exemple 3 : Cis (-) 2-[3-(4-chlorophényl)-2-(E)-propényl]-6-[[2-(2,4-dichlorophényl)-2-(1H-imidazol-1-ylméthyl)-1,3-dioxolan-4-yl]-méthoxy]-1,2,3,4-tétrahydro-isoquinoline.

En opérant comme à l'exemple 2, à partir du méthylphényl sulfonate de cis-(-)-2, (2,4-dichlorophényl)-2-[(1H-imidazol-1-yl)-méthyl]-1,3-dioxolan-4-méthanol et du produit de la préparation 1, on a obtenu le produit recherché. F ≃ 120°C.
α_{D} : -10° (c = 1% méthanol).

### Préparation 2 :

Les produits de départ des exemples 2 et 3 à savoir le méthyl toluène sulfonate de Cis-(+)-2-(2,4-dichlorophényl)-2-(1H-imidazol-1-ylméthyl)-1,3-dioxolane-4-méthanol, et le composé Cis(-) correspondant ont été préparés par chromatographie sur phase chinale à partir du produit Cis ± (I) correspondant.
Isomère Cis (+) α_{D} = + 7°
Isomère Cis (-) α_{D} = - 7°

### Exemple 4 :

On chauffe à 65°C pendant 4 h 30 un mélange de 0,3 mmole de dérivé ArX, 1,5 ml (0,2 mmole) du produit de la préparation 3 et 1,5 ml de solution (0,02 mmole) de PdCl₂(P 3)2 dans le DMF.

On ajoute une solution renfermant de l'eau, du fluorure de potassium, du carbonate acide de sodium (80-10-70). On extrait au chlorure de méthylène et sèche.

On a obtenu ainsi les produits

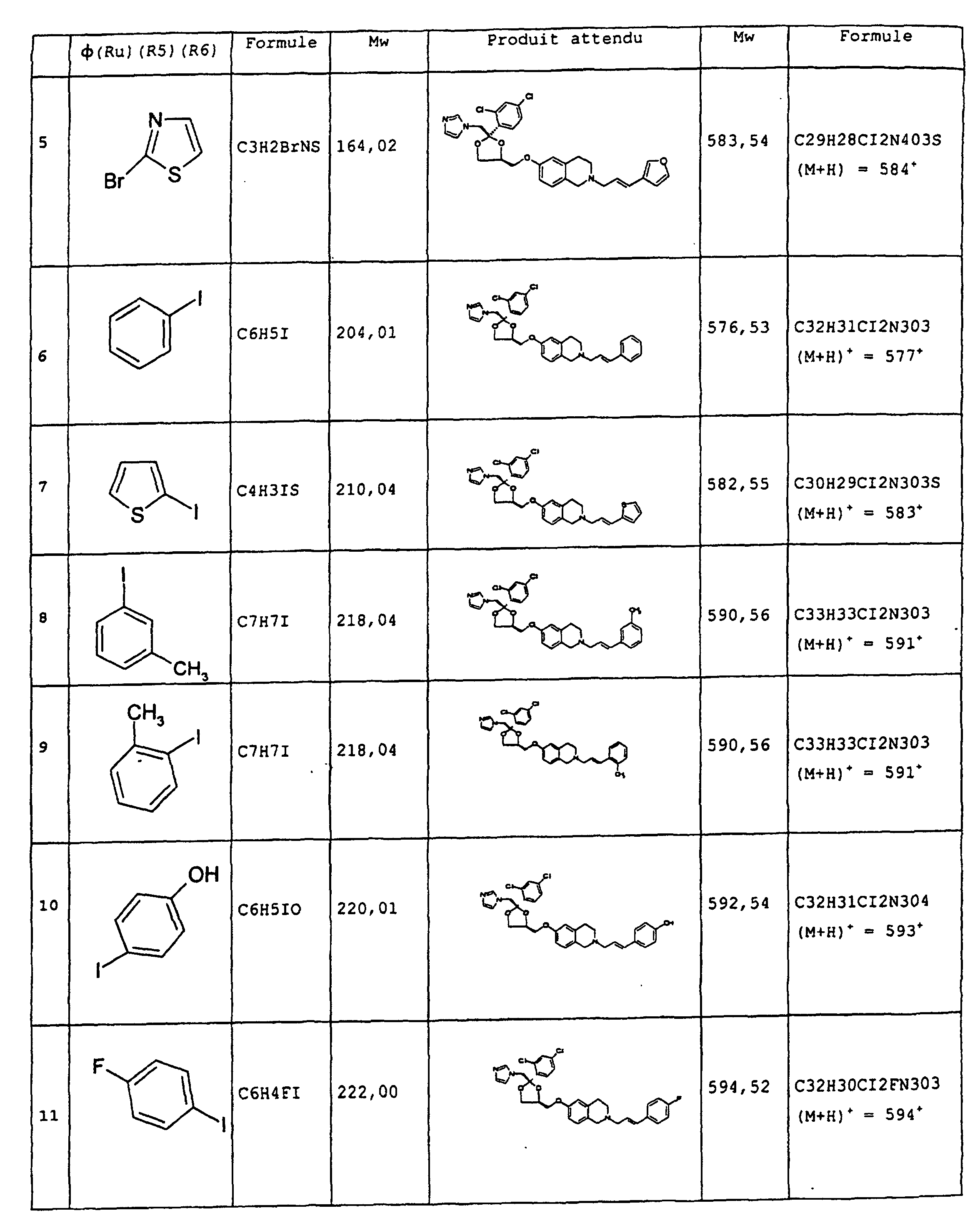

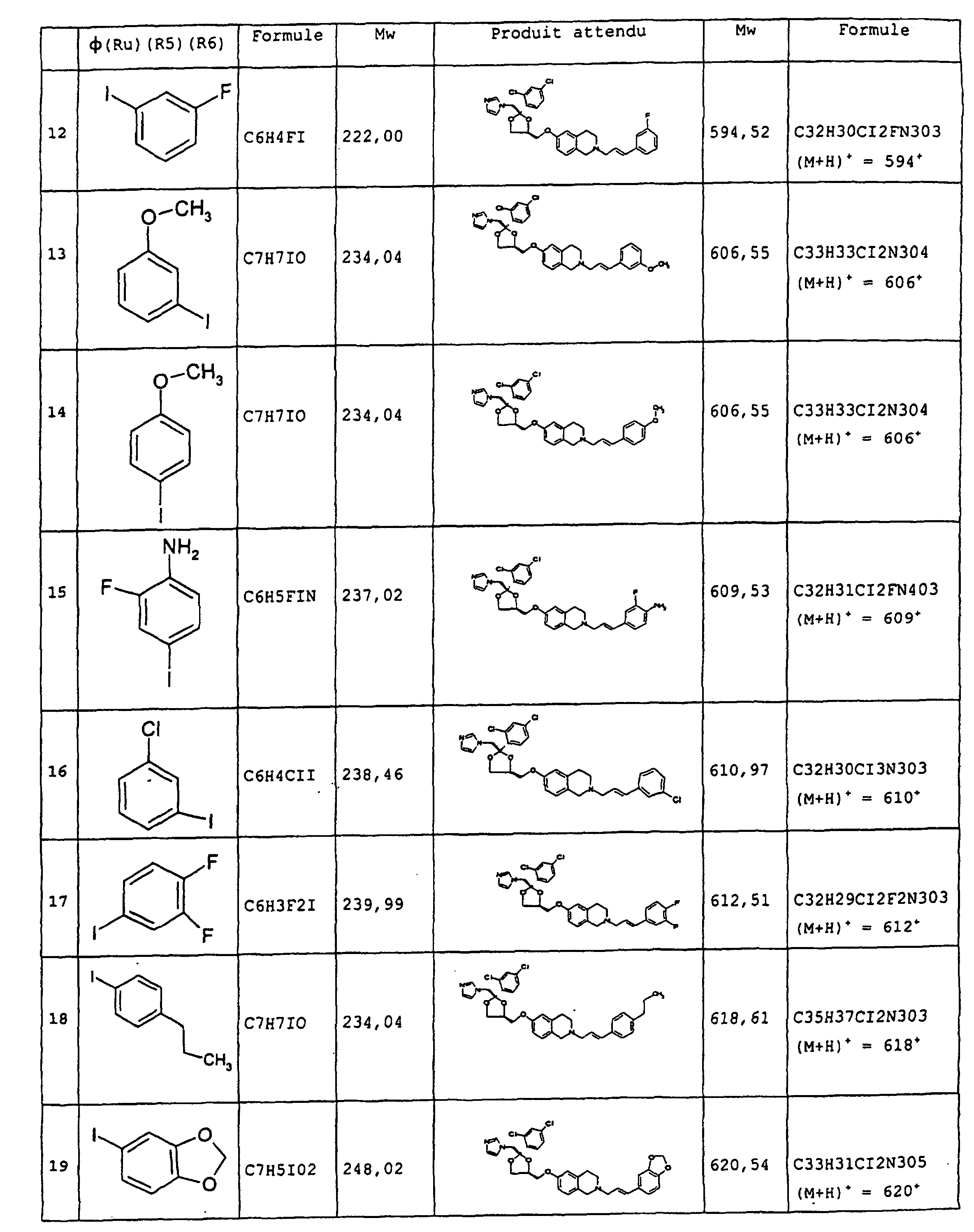

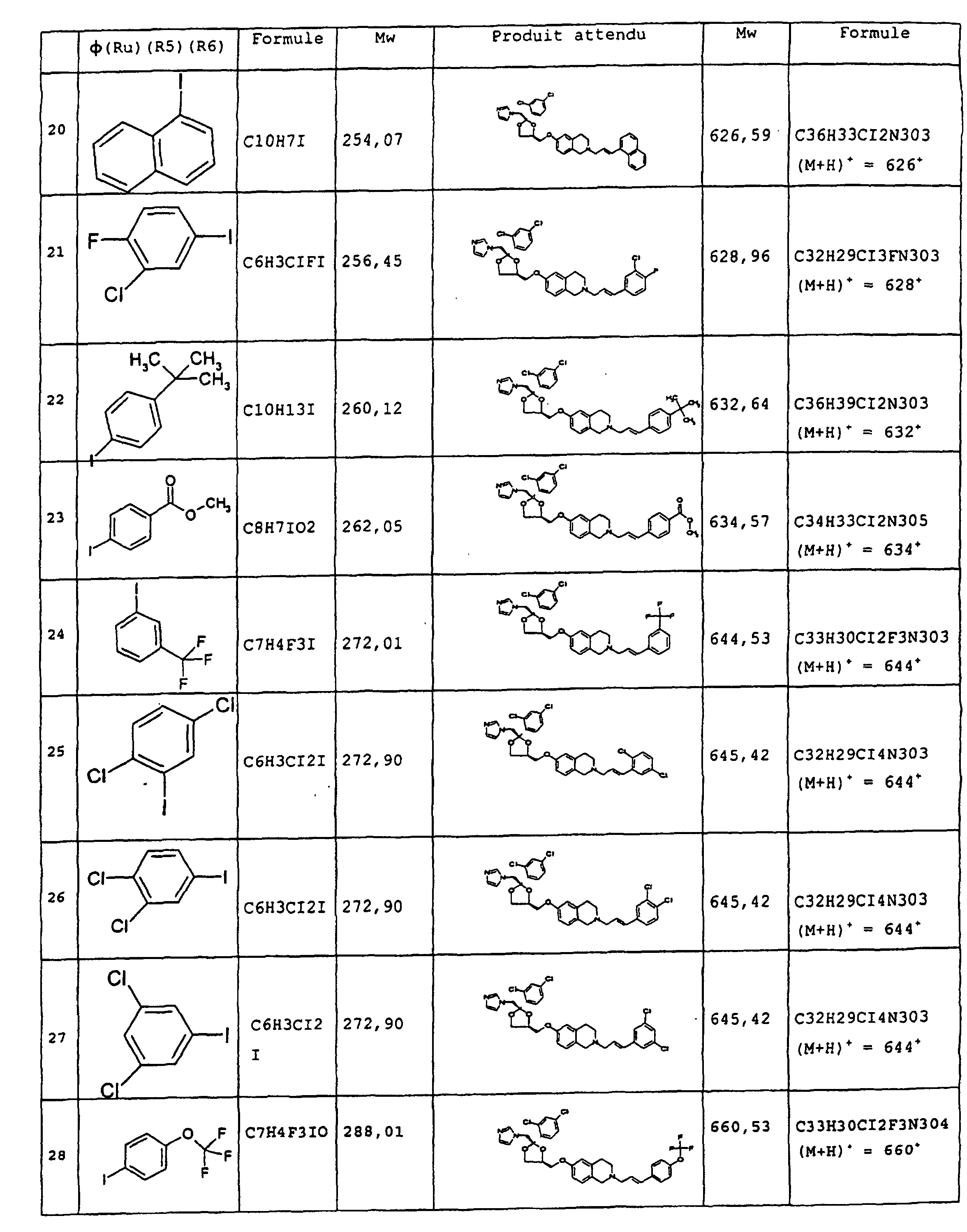

En opérant comme précédemment, on a également préparé les produits suivants : 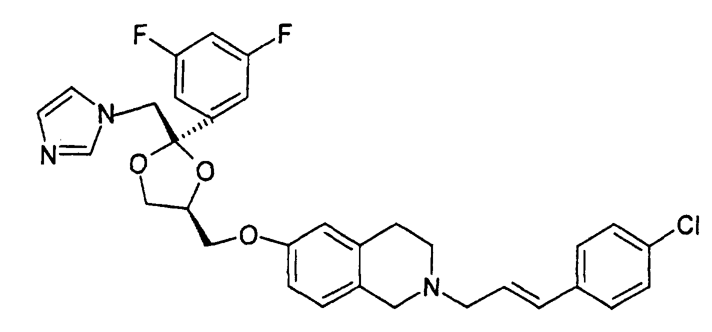 Rf : 0,30 acétate d'éthyle-triéthylamine 95-5

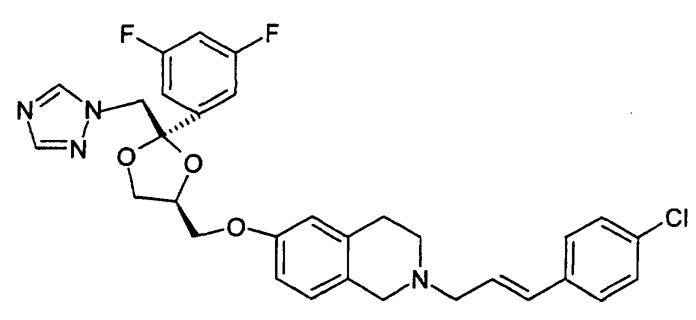

### Préparation 3 : Cis (±) 6-[[2-(2,4 dichlorophényl)-2-(1H-imidazol-1-ylméthyl)-1,3-dioxolan-4-yl]-méthoxy]-1,2,3,4-tétrahydro-2-[3-tributylstannyl)-2(E)-propényl]-isoquinoléine

### Stade A : 3-(tributylstannyl)-2(E)-propen-1-ol

On dissout 12 g de 3-(tributylstannyl)-2(E)-propenoate de methyle dans 100 ml de THF. On refroidit à -78°C, on ajoute 67 ml d'hydrure de dibutylaluminium. On porte la température à 0°C, coule dans le méthanol, ajoute de l'eau et agite une nuit. On filtre les sels d'aluminium, lave à l'acétate d'éthyle, décante les phases organiques, sèche, concentre. On obtient 9 g de produit que l'on purifie par chromatographie en éluant avec le mélange hexane acétate d'éthyle 8/2. On obtient 7,25 g du produit recherché.

### Stade B : (3-bromo-1-(E)-propényl)tributyl-stannane

On coule goutte à goutte en 30 minutes à 0°C une solution de 5,5 g de triphenylphosphine dans 10 ml de chlorure de méthylène dans une solution renfermant 5,2 g de du produit obtenu au stade A dans 50 ml de chlorure de méthylène et 6 g de tétrabromure de carbone. On maintient une heure à 0°C. On verse sur l'eau et extrait au chlorure de méthylène, on sèche, chromatographie sur silice en éluant à l'heptane et obtient 5,06 g de produit recherché.

### Stade C : Cis (±) 6-[[2-(2,4 dichlorophényl)-2-(1H-imidazol-1-yl-méthyl)-1,3-dioxolan-4-yl]-méthoxy]-1,2,3,4-tétrahydro-2-[3-tributylstannyl)-2(E)-propényl]-isoquinoléine.

On coule goutte à goutte sous agitation et atmosphère d'azote une solution de 5,06 g de produit préparé précédemment et 20 ml d'acétone, dans un mélange comprenant 5,68 g de Cis (±) 6-[[2-(2,4-dichlorophényl)-2-(1H-imidazol-1-yl-méthyl)-1,3-dioxolan-4-méthyl]-méthoxy]-1,2,3,4-tétrahydro-isoquinoléine préparé à la préparation 4, 1,5 g d'Ag2, 100 ml d'acétone et 50 ml de DMF. On maintient une nuit à la température ambiante. On filtre, reprend à l'eau, extrait à l'acétate d'éthyle, purifie par chromatographie sur silice (éluant : chlorure de méthylène, méthanol (95-5). On obtient 6,66 g de produit recherché.

### PREPARATION 4 : Cis (±) 6-[[2-(2,4 dichlorophényl)-2-(1H-imidazol-1-yl-méthyl)-1,3-dioxolan-4-yl]-méthoxy]-1,2,3,4-tétrahydro-isoquinoléine

### Stade A :

On agite pendant 15 heures à la température ambiante une suspension renfermant 5,8 g de 1,2,3,4 tétrahydroisoquinoléine 6-OH et 50 ml de THF. On ajoute à 20°C, 11,13 g de diterbutyl dicarbonate dans 25 ml de THF. On verse dans une solution glacée de carbonate acide de potassium. On extrait à l'acétate d'éthyle. On sèche. On filtre et concentre. On reprend le produit obtenu dans le pentane, amorce et lave les cristaux obtenus au pentane. On obtient 9,26 g de produit recherché F = 114°C.

### Stade B :

On introduit en 20 minutes à la température ambiante dans une solution de 24,63 g du produit préparé en stade A et 250 ml de DMF, 5,4 g d'hydrure de sodium à 55 ≅ 60% en dispersion dans l'huile. On porte à 55°C pendant 2 heures. On laisse revenir à la température ambiante et introduit 54,6 g de méthane sulfonate de cis-(+)-2-(2,4-dichlorophényl)-2-(1H-imidazol-1-yl-méthyl)-1,3-dioxolane-4-méthanol. On porte le mélange réactionnel à 80°C pendant 20 heures. On agite ensuite à la température ambiante pendant 72 heures. On verse sur de la glace, agite une heure, décante, sèche, filtre et concentre. On obtient 95,8 g de produit que l'on chromatographie sur silice en éluant avec le mélange heptane acétone (6/4). On obtient ainsi 45 g de produit recherché que l'on utilise tel quel dans le stade suivant.

### Stade C : Cis (±) 6-[[2-(2,4 dichlorophényl)-2-(1H-imidazol-1-yl-méthyl)-1,3-dioxolan-4-yl]-méthoxy]-1,2,3,4-tétrahydroisoquinoléine.

On refroidit à 10°C, 45 g du produit préparé au stade précédent en solution dans 200 cm³ d'acétate d'éthyle. On ajoute 100 cm³ d'une solution renfermant 50 g de glace et 50 cm³ d'une solution acide chlorhydrique 12N. On agite le mélange réactionnel pendant 4 heures. On concentre sous pression réduite. On reprend à l'acétate d'éthyle. On obtient un produit que l'on reprend dans l'éther éthylique. On triture le produit obtenu, essore, rince et sèche. On obtient 60,8 g de produit que l'on verse dans 250 cm³ d'eau. On refroidit et coule 50 cm³ d'une solution d'ammoniaque à 28%. On agite une demi-heure et ajoute 150 cm³ de chlorure de méthylène. On maintient le mélange réactionnel sous agitation pendant 30 minutes, extrait au chlorure de méthylène, lave, sèche, filtre et concentre. On obtient 35,68 g de produit recherché.

### Compositions pharmaceutiques

On a préparé des composés renfermant

| | |
|---|---|
| Produit de l'exemple 1 | .50 mg |
| Excipient q.s.p. | 1 g |

Détail de l'excipient: amidon, talc, stéarate de magnésium.

### Activité biologique

Activité antifongique du produit de l'exemple 1 ou produit P. On utilise des souris femelles pesant de 18 à 22 g. On leur administre dans la veine de la queue une quantité de Candida Albicans 44858 à raison de 10⁶CFU par souris (CFU : unité formant des colonies). On sépare les souris en 5 lots de 5 souris et on les traite de la façon suivante :
Une heure après l'infection
- groupe 1 : les souris sont traitées avec le produit P 25mg/kg par voie orale
- groupe 2 : les souris sont traitées avec le produit P par voie intrapéritonéale à raison de 25mg/kg
- groupe 3 : les souris sont traitées avec le fluconazole (25mg/kg par voie orale).
- groupe 4 : les souris sont traitées avec le fluconazole (25mg/kg par voie intrapéritonéale).
- groupe 5 : les souris ne reçoivent aucun traitement antifongique.

Pendant une période de 22 jours, on compte les souris mortes.

### Conclusion

Le produit à la dose utilisée dans les 2 modes d'administration utilisés présente une excellente activité. De plus l'essai a été effectué avec une administration sous ordre que ceux obtenus avec le fluconazole.

Les mêmes traitements sont également efficaces dans le "modèle topique" avec les fungis dermiques par exemple trichophyton et dans le modèle sublétale.

### Concentration minimale inhibitrice (CMI)

Des cellules de Candida albicans sont préparées comme indiqué dan Journal of Antimicrobial chemotherapy 38, 579-587, lavées 3 fois avec une solution 0,1 M de phosphate et utilisées immédiatement pour déterminer la concentration minimale inhibitrice (CMI).

Les CMI sont déterminés par la modification d'une plaque microtitré selon la méthode standard du Comité National des standards cliniques de laboratoire.

On utilise comme milieu RPMI-1640, et de la L-glutamine tamponnée à pH7 avec une solution 0,15 M de MOPS (acide 3-[N-morpholino]propane sulfonique). On ajoute les cellules de Candida albicans (1,5 X 10³ cellules/ml) dans les puits d'une plaque de 96 puits contenant RPMI-1640 et les dilutions d'agents antifongiques. On fait la lecture des résultats 48 heures après incubation à 35°C et on détermine la CMI ou concentration minimale inhibitrice qui inhibe la croissance des cellules du Candida albicans.

### Concentration minimale fongicide

Après la lecture à 48 heures des CMI, on secoue les plaques et retire 10µL d'aliguot des puits que l'on place sur des disques rectangulaires contenant du dextrose agar. Les plaques sont incubées pendant 48 heures à 35°C ; La concentration minimale fongicide et la concentration de l'agent antifongique à laquelle le nombre d'unité formant des colonies est zéro.

## Revendications

1. Sous toutes les formes stéréoisomères possibles ainsi que leur mélange, les composés de formule (I) 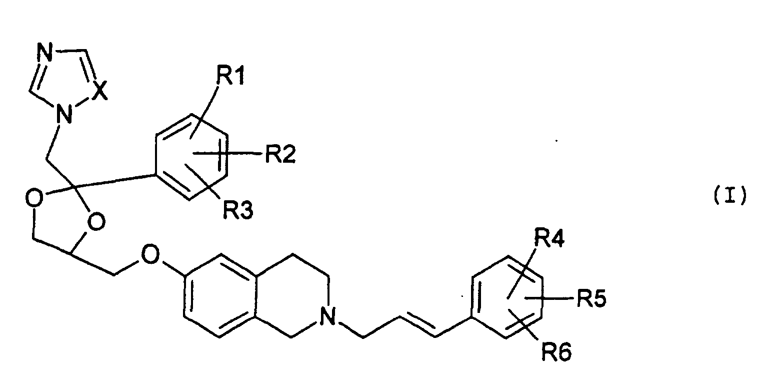 dans laquelle X représente un atome d'azote ou un radical -CH ≃ R1, R2, R3, R4, R5, R6 identiques ou différents en position quelconque sur les cycles qui les portent représentent un atome d'hydrogène, un atome d'halogène, un radical alkyle, O-alkyle, 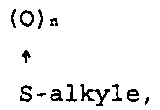 alkényle, O-alkényle, 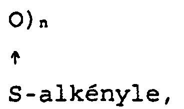 alkynyle, O-alkynyle, 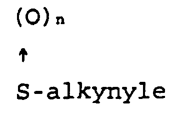 renfermant jusqu'à 8 atomes de carbone, éventuellement substitué par un ou plusieurs atomes d'halogène, n représentant le nombre 0, 1 ou 2, ou représentent un radical NO₂, NH₂ ou C≡N, R1, R2, R3, R4, R5 et R6 pouvant former des cycles deux à deux, ainsi que leurs sels d'addition ou avec les acides.

2. Les composés définis à la revendication 1, répondant à la formule (IA) 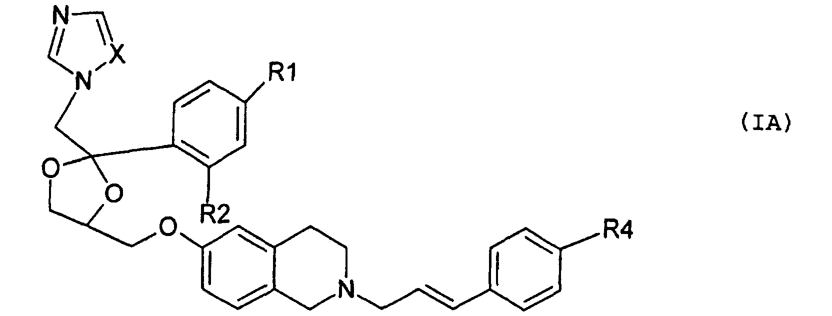 dans lesquels X, R1 R2 et R4 conservent leur signification précédente.

3. Les composés de formule(I) et (IA) définis à l'une quelconque des revendications 1 et 2 dans lesquels, X représente un radical -CH=.

4. Les composés de formule (I) et (IA) définis à l'une des revendications 1 à 3 dans lesquels R1 et R2 représentent chacun un atome d'halogène.

5. Les composés de formule(I) et (IA) définis à l'une quelconque des revendications 1 à 4 dans lesquels R4 représente un atome d'halogène.

6. Les composés de formule (I) et (IA) définis à l'une quelconque des revendications 1 à 4 dans lesquels R1, R2 et R4 représentent un atome de chlore.

7. Le composé de formule (I) suivant : La Cis-(±)-2-[3-(4-chlorophényl)-2-(E)-propényl]-6-[[2-(2,4-dichlorophényl)-2-(1H-imidazol-1-ylméthyl)-1,3-dioxolan-4-yl]méthoxy]-1,2,3,4-tétrahydro-isoquinoléine.

8. L'énantiomère cis (+) du produit de la revendication 7

9. Procédé de préparation des composés de formule (I) définis à l'une quelconque des revendications 1 à 7 **caractérisé en ce que** l'on soumet un composé de formule (II) 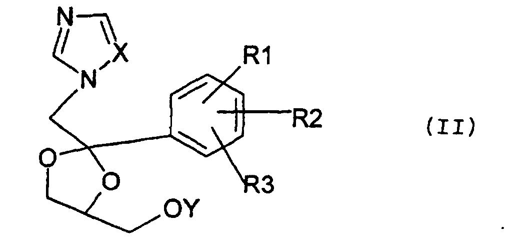 dans laquelle
Y représente un radical mésyle ou tosyle
et les autres substituants conservent leur signification précédente à l'action d'un composé de formule (III) 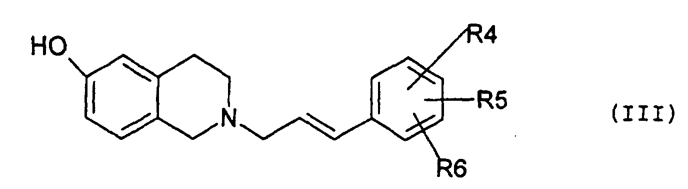 dans laquelle les différents substituants conservent leur précédente signification pour obtenir le composé de formule (I) correspondant.

10. A titre de produits chimiques nouveaux les composés de formule (III) définis à la revendication 9.

11. Procédé de préparation des composés de formule I défini à la revendication 1, **caractérisé en ce que** l'on soumet un composé de formule (IV) 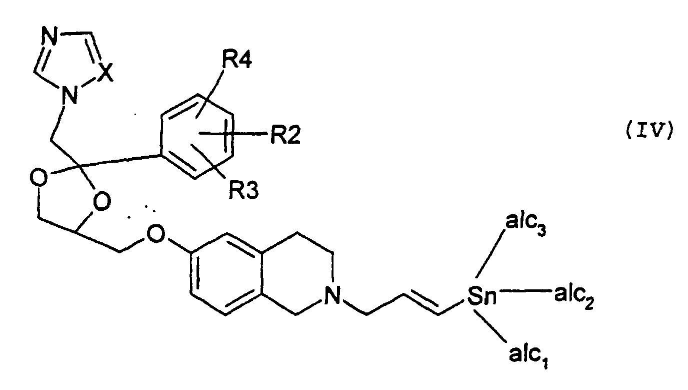 dans laquelle alc₁, alc₂ et alc₃ représentent un radical alcoyle refermant jusqu'à 8 atomes de carbone, dans laquelle R₁, R₂, R₃ et X conservent leur signification précédente, à l'action d'un composé de formule (V) 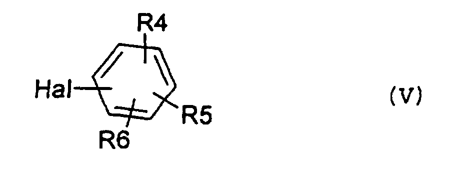 dans laquelle R₄, R₅ et R₆ conservent leur signification précédente et Hal représente un atome d'halogène, pour obtenir le composé de formule (I) correspondant.

12. Les compositions antifongiques renfermant comme principe actif au moins un composé défini à l'une quelconque des revendications 1 à 7.

13. A titre de médicaments, les composés de formule (I) définis à l'une quelconque des revendications 1 à 7 ainsi que leurs sels d'addition avec les acides pharmaceutiquement acceptables.

## Claims

1. The compounds of formula (I)in all possible stereoisomer forms as well as their mixtures, 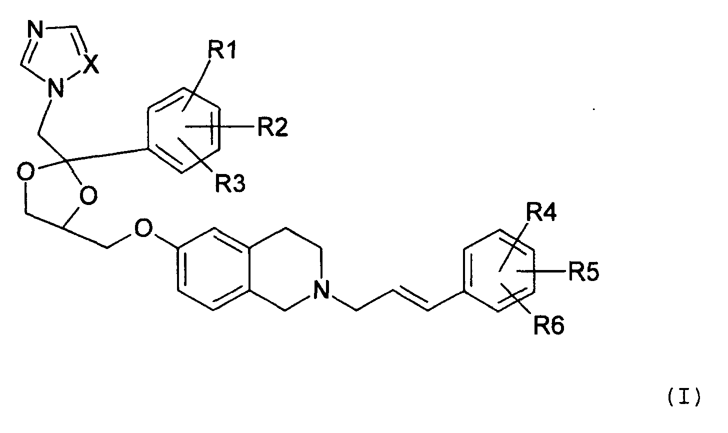 in which X represents a nitrogen atom or an identical or different a -CH = radical R1, R2, R3, R4, R5, R6 in any position on the rings which carry them represent a hydrogen atom, a halogen atom, an alkyl, O-alkyl, 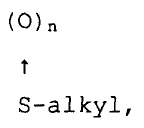 alkenyl, O-alkenyl, 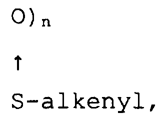 alkynyl, O-alkynyl, 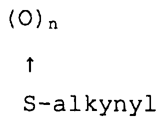 radical containing up to 8 carbon atoms, optionally substituted by one or more halogen atoms, n representing the number 0, 1 or 2, or represent an NO₂, NH₂ or C≡N radical, R1, R2, R3, R4, R5 and R6 able to form rings in pairs, as well as their addition salts or with acids.

2. The compounds defined in claim 1, corresponding to formula (IA) 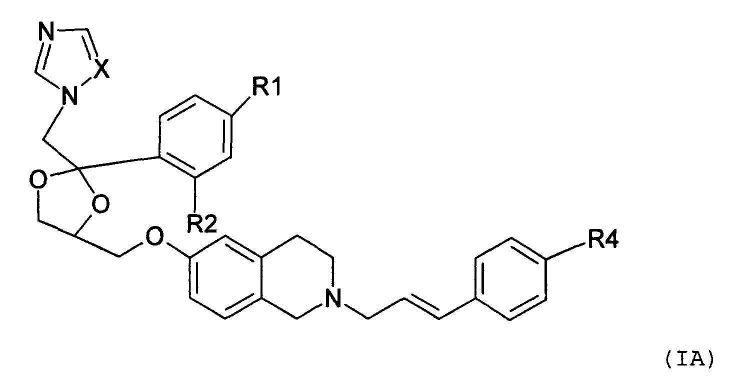 in which X, R1 R2 and R4 retain their previous meaning.

3. The compounds of formula(I) and (IA) defined in any one of claims 1 and 2 in which, X represents a -CH= radical.

4. The compounds of formula (I) and (IA) defined in one of claims 1 to 3 in which R1 and R2 each represent a halogen atom.

5. The compounds of formula(I) and (IA) defined in any one of claims 1 to 4 in which R4 represents a halogen atom.

6. The compounds of formula (I) and (IA) defined in any one of claims 1 to 4 in which R1, R2 and R4 represent a chlorine atom.

7. The following compound of formula (I): Cis-(±)-2-[3-(4-chlorophenyl)-2-(E)-propenyl]-6-[[2-(2,4-dichlorophenyl)-2-(1H-imidazol-1-ylmethyl)-1,3-dioxolan-4-yl]methoxy]-1,2,3,4-tetrahydro-isoquinoline.

8. The cis (+) enantiomer of the product of claim 7

9. Process for the preparation of the compounds of formula (I) defined in any one of claims 1 to 7 **characterized in that** a compound of formula (II) 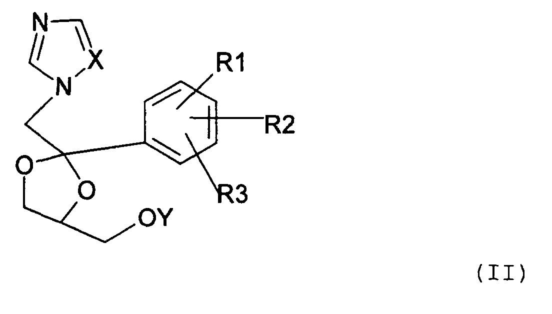 in which
Y represents a mesyl or tosyl radical
and the other substituents retain their previous meaning is subjected to the action of a compound of formula (III) 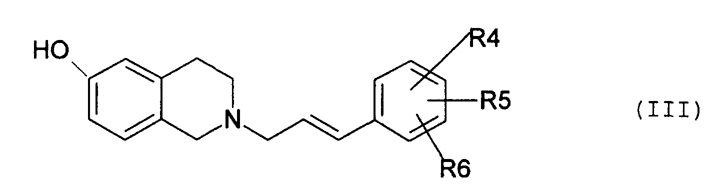 in which the different substituents retain their previous meaning in order to obtain the corresponding compound of formula (I).

10. As new chemical products the compounds of formula (III) defined in claim 9.

11. Process for the preparation of the compounds of formula I defined in claim 1, **characterized in that** a compound of formula (IV) 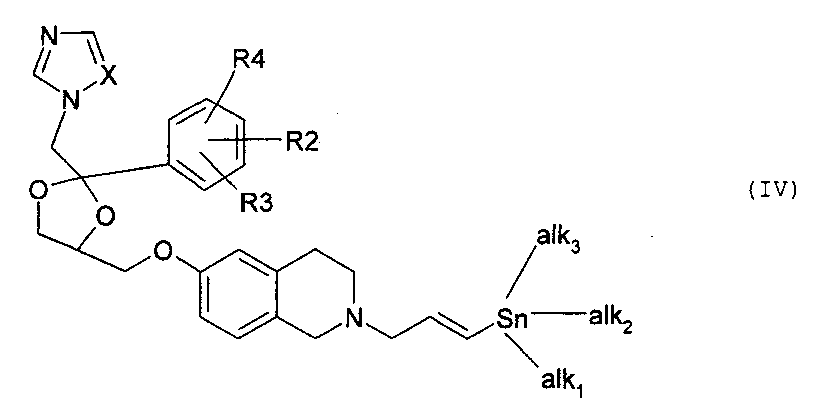 in which alk₁, alk₂ and alk₃ represent an alkoyl radical containing up to 8 carbon atoms, in which R₁, R₂, R₃ and X retain their previous meaning, is subjected to the action of a compound of formula (V) 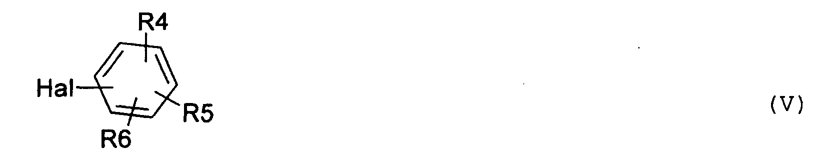 in which R₄, R₅ and R₆ retain their previous meaning and Hal represents a halogen atom, in order to obtain the corresponding compound of formula (I).

12. Antifungal compositions containing as active ingredient at least one compound defined in any one of claims 1 to 7.

13. As medicaments, the compounds of formula (I) defined in any one of claims 1 to 7 as well as their addition salts with pharmaceutically acceptable acids.

## Patentansprüche

1. In allen möglichen stereoisomeren Formen sowie ihren Mischungen die Verbindungen der Formel (I) 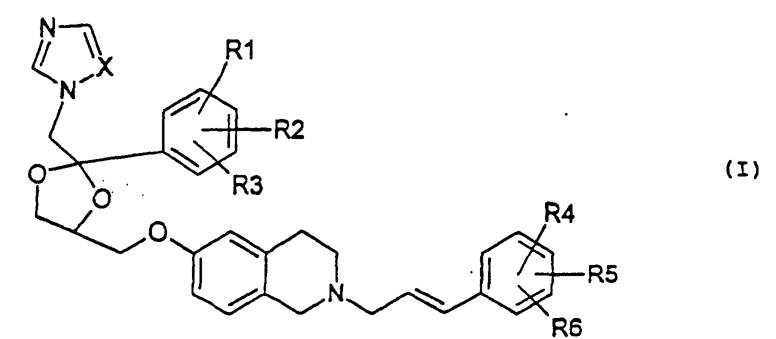 in der X ein Stickstoffatom oder einen Rest -CH= darstellt, R1, R2, R3, R4, R5, R6, gleich oder verschieden, in irgendeiner Position an den Ringen, die sie tragen, bedeuten: ein Wasserstoffatom, ein Halogenatom, einen Rest Alkyl, O-Alkyl, 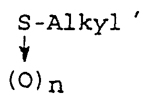 Alkenyl, O-Alkenyl oder 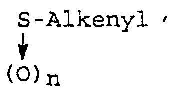 Alkinyl, O-Alkinyl oder 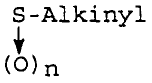 mit bis zu 8 Kohlenstoffatomen, gegebenenfalls substituiert durch ein oder mehrere Halogenatome, und n die Zahl 0, 1 oder 2 ist, oder einen Rest NO₂, NH₂ oder C≡N darstellen, wobei R1, R2, R3, R4, R5 und R6 paarweise Ringe bilden können, sowie ihre Additionssalze mit Säuren.

2. Verbindungen wie in Anspruch 1 definiert, die der allgemeinen Formel (IA) 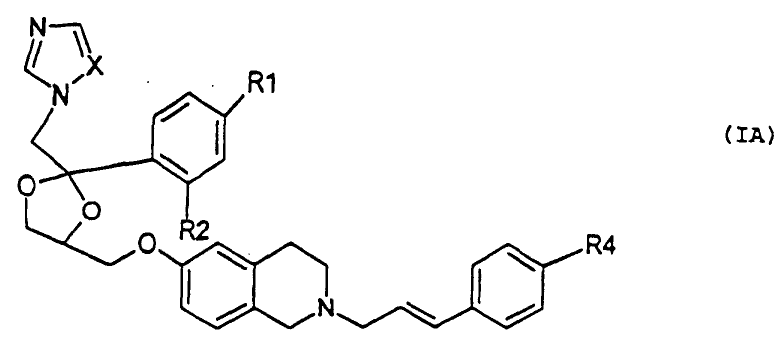 entsprechen, in der X, R1, R2 und R4 ihre vorstehende Bedeutung beibehalten.

3. Verbindungen der Formeln (I) und (IA) wie in irgendeinem der Ansprüche 1 und 2 definiert, in denen X einen Rest -CH= bedeutet.

4. Verbindungen der Formeln (I) und (IA) wie in irgendeinem der Ansprüche 1 bis 3 definiert, in denen R1 und R2 jeweils ein Halogenatom bedeuten.

5. Verbindungen der Formeln (I) und (IA) wie in irgendeinem der Ansprüche 1 bis 4 definiert, in denen R4 ein Halogenatom bedeutet.

6. Verbindungen der Formeln (I) und (IA) wie in irgendeinem der Ansprüche 1 bis 4 definiert, in denen R1, R2 und R4 ein Chloratom bedeuten.

7. Die Verbindung der Formel (I), die folgt: cis-(±)-2-[3-(4-Chlorphenyl)-2-(E)-propenyl]-6-{[2-(2,4-dichlorphenyl)-2-(1H-imidazol-1-yl-methyl)-1,3-dioxolan-4-yl]-methoxy}-1,2,3,4-tetrahydro-isochinolin.

8. Das cis(+)-Enantiomer des Produktes von Anspruch 7.

9. Verfahren zur Herstellung der Verbindungen der Formel (I) wie in irgendeinem der Ansprüche 1 bis 7 definiert, **dadurch gekennzeichnet, daß** man eine Verbindung der Formel (II) 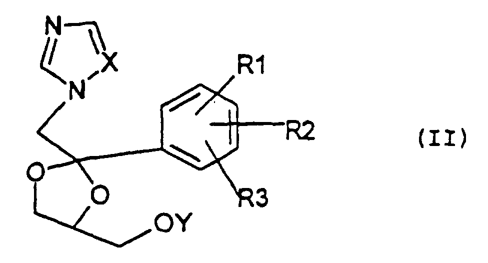 in der
Y einen Rest Mesyl oder Tosyl darstellt und die anderen Substituenten ihre vorstehend genannte Bedeutung beibehalten, der Einwirkung einer Verbindung der Formel (III) 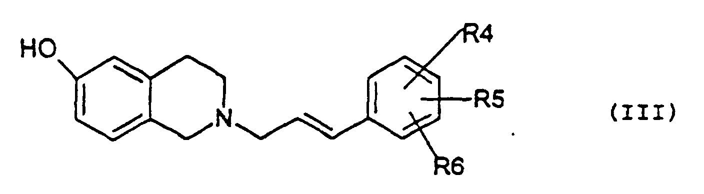 unterzieht, in der die verschiedenen Substituenten ihre vorstehend genannte Bedeutung beibehalten, um die entsprechende Verbindung der Formel (I) zu erhalten.

10. Als chemisch neue Produkte die Verbindungen der Formel (III) wie in Anspruch 9 definiert.

11. Verfahren zur Herstellung der Verbindungen der Formel (I) wie in Anspruch 1 definiert, **dadurch gekennzeichnet, daß** man eine Verbindung der Formel (IV) 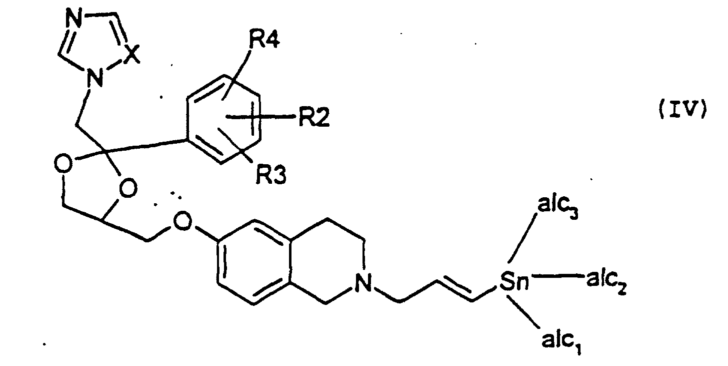 in der alc₁, alc₂ und alc₃ einen Rest Alkyl mit bis zu 8 Kohlenstoffatomen darstellen und in der R1, R2, R3 und X ihre vorstehende Bedeutung beibehalten, der Einwirkung einer Verbindung der Formel (V) 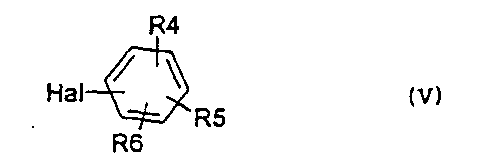 unterzieht, in der R4, R5 und R6 ihre vorstehende Bedeutung beibehalten und Hal ein Halogenatom darstellt, um die entsprechende Verbindung der Formel (I) zu erhalten.

12. Antifungische Zusammensetzungen, umfassend als Wirkstoff mindestens eine Verbindung wie in irgendeinem der Ansprüche 1 bis 7 definiert.

13. Als Arzneimittel die Verbindungen der Formel (I) wie in irgendeinem der Ansprüche 1 bis 7 definiert sowie ihre Additionssalze mit pharmazeutisch akzeptablen Säuren.
